# EUROPEAN PATENT APPLICATION

(11) **EP 4 759 299 A1**
(43) Date of publication of application: **17.06.2026**
(21) Application number: 24220085.5
(22) Date of filing: 16.12.2024
(51) Int. Cl.: A61K 9/00, A61K 9/51, A61K 31/65, A61P 1/02

(54) **ZINC-MAGNESIUM LAYERED HYDROXIDE NANOPARTICLES FOR TREATING PERIODONTITIS AND/OR PERI-IMPLANTITIS AND A METHOD TO OBTAIN THE SAME**

(30) Priority: 12.12.2024 PT 2024119901
(71) Applicant: Universidade Do Porto, 4099-002 Porto (PT); Instituto Politécnico de Setúbal, 2910-761 Setúbal (PT); FARM-ID - ASSOCIAÇÃO DA FACULDADE DE FARMÁCIA PARA A INVESTIGAÇÃO E DESENVOLVIMENTO, 1649-003 Lisboa (PT); REQUIMTE - Rede de Química e Tecnologia, 4050-453 Porto (PT)
(72) Inventor: Martin, Zacharias, 4200-393 Porto (PT); Simao de Campos Bettencourt, Ana, 1649-003 Lisboa (PT); Ferreira dos Santos, Catarina, 2910-761 Setúbal (PT); de Sousa Gomes, Pedro, 4200-393 Porto (PT)
(74) Representative: Couto, Cláudia

(57) **Abstract**

The present application relates to zinc-magnesium layered hydroxide nanoparticles as a drug delivery system for the treatment of periodontitis and/or peri-implantitis. The nanoparticles herein described have a layered hydroxide structure, have enhanced cell interaction and uptake, are biodegradable, efficient at drug loading, have an improved, pH-responsive drug release profile, and enhanced biological efficacy. The application also discloses a method to obtain the zinc-magnesium layered hydroxide nanoparticles.

## Description

### Technical field

This application relates to zinc-magnesium layered hydroxide nanoparticles as a drug delivery system for treating periodontitis and/or peri-implantitis and a method to obtain the same.

### Background art

Periodontitis and peri-implantitis are multifactorial diseases initiated by the presence of periodontal pathogens at the gingival crevice and peri-implant areas, which trigger a host immune response leading to the destruction of the supporting structures. These complex conditions unfold through a dynamic interplay between the bacterial biofilms and the host's immune defenses, leading to the release of pro-inflammatory cytokines, enzymes, and other mediators that contribute to the breakdown of the periodontal connective tissues. The disease's intricacy is compounded by genetic, environmental, and lifestyle factors, emphasizing the necessity for advanced, targeted therapies capable of stopping the tissue destruction that leads to tooth/implant loss and contributes to the systemic inflammation. Standard treatment is set on mechanical scaling and root planning, supplemented by systemic antibiotic therapy in selected conditions. However, this approach often bears the risk of adverse effects and limited therapeutic benefits. Alternatively, the local antibiotic administration at affected sites has been attempted, yet, with a limited success, primarily due to a rapid clearance and low substantivity of the therapeutic agents.

In response to these limitations, controlled local drug delivery strategies have been introduced, such as Atridox, Elyzol, Actisite, Arestin and PerioChip. These systems encompass the combination of distinctive carrier materials with active pharmacological agents to enhance and sustain therapeutic action at specific sites, thereby reducing the potential systemic adverse effects and protecting the drug from degradation. The carriers, comprising a range of macro and microscale materials such as antibiotic-laden hydrogels, polymeric fibers and microparticles have been specifically designed to the local application into periodontal pockets. These products were devised to provide a sustained drug release within the targeted environment, significantly decreasing the necessity for frequent intrapocket applications, enhancing patient convenience, and fostering adherence to treatment protocols.

Despite these advancements, existing models suffer from limitations, including their intricate placement, often conditioned on the depth of the periodontal pocket, and the difficulty in achieving a consistent and effective drug release. Carrier's misplacements, inconsistent resorption, and unsubstantiated release profile may contribute to inconclusive data regarding their effectiveness in preventing further surgeries, long-term beneficial effects, and cost-effectiveness. Thus, the combination of these drawbacks, along with the questionable effectiveness of these systems against internalized bacteria, possibly hindered their widespread clinical adoption.

Currently, nanoparticle-based alternatives for periodontitis and/or peri-implantitis treatment are not commercially available, despite the substantial number of patent documents on this topic. These patents include distinct polymeric and metal-oxide nanoparticles (NPs), loaded with antibacterial drugs and anti-inflammatory compounds that can be incorporated into external matrices as gels. Nonetheless, neither of these systems describe intracellular activity nor nanoparticles set on complex structures such as layered hydroxides, which hold promise for local drug delivery due to their intrinsic characteristics. Additionally, most systems describe complex synthesis processes, involving numerous steps.

### Summary

The present invention relates to zinc-magnesium layered hydroxide nanoparticles as a drug delivery system loaded with at least one type of hydrophilic therapeutic agent for the treatment of periodontitis and/or peri-implantitis, wherein the nanoparticles have a structure made of hydroxide layers comprising an interlayer spacing between 9.5 to 10 Å, a layer thickness between 0.4 and 0.7 nm, and a positive surface charge between 15 and 40mV.

In one embodiment, the hydrophilic therapeutic agent is a tetracycline antibiotic.

In one embodiment, the ratio between nanoparticles:therapeutic agent ranges between 5.7:1 and 13:1 weight.

In one embodiment, the drug loading of the hydrophilic therapeutic agent ranges between 7 and 15% in weight.

In another embodiment, the invention relates to a method to obtain the zinc-magnesium layered hydroxide nanoparticles, wherein it comprises the following steps:
- Preparing a zinc nitrate aqueous solution with a weight concentration between 25 to 32%;
- Preparing a magnesium nitrate aqueous solution with a weight concentration between 2 to 3%;
- Adding at least one type of hydrophilic therapeutic agent to the magnesium nitrate aqueous solution under stirring and a temperature between 20 and 25°C, for at least 5 minutes, obtaining a concentration between 1 to 5 mg/mL of the agent;
- Adding the zinc nitrate aqueous solution to the previous solution, obtaining a mixture with a Zn:Mg ratio ranging from 9:1 to 11:1 M;
- Adjusting the pH of the mixture between 1.5 and 2.5;
- Adding a precipitation agent dropwise to the mixture under stirring and temperature between 20 and 25°C, for at least 10 minutes;
- Adjusting the pH of the mixture between 5 and 6;
- Filtering the mixture under vacuum at a temperature between 20 and 25°C to obtain a precipitate;
- Washing the precipitate with ultra-pure water and drying the precipitate at a temperature between 20 and 25°C under vacuum for at least 24 h to obtain the nanoparticles loaded with the therapeutic agent.

In one embodiment, the zinc nitrate aqueous solution is obtained by dissolving zinc nitrate hexahydrate in ultra-pure water, under stirring 300 RPM at a temperature between 20 and 25°C, for at least 5 minutes.

In one embodiment, the magnesium nitrate aqueous solution is obtained by dissolving magnesium nitrate hexahydrate in ultra-pure water, under stirring 300 RPM at a temperature between 20 and 25°C, for at least 5 minutes.

In one embodiment, the precipitation agent is ammonia at 25%.

In one embodiment, the stirring is carried out between 300 to 900 RPM.

In one embodiment, the filtering step is carried out with a 0.22 µm filter membrane.

### General description

In contrast to the previously described auxiliary therapies for periodontitis/peri-implantitis treatment, the present invention introduces a uniquely nanostructured drug delivery system. Setting on an innovative one-step synthesis to produce nanoparticles (NPs) comprising therapeutic agents, the technical enhancements introduced by this invention potentially elevate nanoparticle's functional capabilities and clinical effectiveness, including:
- **Enhanced cell interaction and uptake:** The developed nanoparticles, due to their size, physicochemical and surface properties, are designed to foster interactions with eukaryotic cells, avoiding particle's opsonization, and enhancing cellular uptake. This capability is crucial for modulating intracellular biological processes and effectively targeting intracellular bacteria, which are often challenging to reach and are key contributors to disease recurrence, thus substantially improving therapeutic outcomes.
- **Biodegradable and bioactive composition:** The distinctive incorporation of zinc and magnesium into the nanoparticles' composition not only endorses biological functionality, given the elements relevance for physiological human homeostasis, but further confers biodegradability and bioactivity to the system. The zinc-magnesium nanoparticles can induce oxidative damage and disturb bacterial cell walls, thereby enhancing the efficacy of the therapeutic agents through synergistic interactions.
- **Layered hydroxide structure:** A distinctive feature of these nanoparticles is their layered hydroxide structure, characterized by cationic layers interspersed with anionic molecules within the interlayer spaces. This architecture facilitates the strategic substitution of interlayer anionic molecules with therapeutic agents, offering an adjustable interlayer distance that enhances drug-loading capacity beyond that of traditional nanoparticles.
- **Eco-friendly synthesis:** The synthesis method eschews the use of organic solvents and high-temperature techniques frequently employed to produce conventional polymeric and ceramic particles, addressing environmental concerns, and reducing energy consumption and the risks posed by solvent emissions and usage.
- **Efficient drug-loading:** The method allows for the incorporation of water-soluble therapeutic agents during synthesis, merging the drug loading stage with the carrier synthesis, into a streamlined, scalable process, with lower error summation and high reproducibility. Conventional drug loading techniques such as ionic gelation and single emulsion, employed for organic particles, broadly present poor results regarding loading and release efficiency for water-soluble drugs. Therefore, this synthesis method allows a seamlessly loading with hydrophilic drugs with enhanced drug loading capacity.
- **Improved drug release profile:** The invention stands out for its ability to achieve an optimal therapeutic agent release profile, ensuring that the agent is released in a targeted manner, aligned with the clinical needs of periodontal treatment.
- **pH-responsive drug release:** The rate at which the therapeutic agent is released can be modulated by the pH of the surrounding environment. Acidic environments, which mimic those found in inflamed and infected tissues, accelerates the release of the agent, thereby increasing its local concentration and potentially improving therapeutic efficacy.
- **Demonstrated superior biological efficacy:** Preliminary *in vitro* biological evaluations demonstrate that the invention presents anti-inflammatory, ROS scavenging, and antibacterial activities at cytocompatible concentrations, underscoring its potential to significantly push forward periodontitis and peri-implantitis therapy.

The paramount technical advantage of this invention stands on its innovative synthesis method that yields a nanoscale system with a unique layered hydroxide structure, facilitating high drug loading and optimized release. This nanoscale configuration improves cellular uptake, directly delivering therapeutic agents to intracellular targets, including hard-to-reach bacteria, and modulating the inflammatory response, pivotal in periodontitis and/or peri-implantitis treatment. By combining bacterial targeting and inflammation modulation within a single integrated approach, this system marks a significant leap forward in periodontal therapy, offering a comprehensive solution that addresses both the symptoms and the underlying causes of periodontal disease in a highly effective manner.

As an unexpected effect, the presence of magnesium in the synthesis appears to influence the resulting product, favoring zinc layered hydroxide over zinc oxide particles. Furthermore, the conjugation with a hydrophilic therapeutic agent with the carrier did not hinder its antibacterial and pleiotropic effects.

Doxycycline hydrochloride, a widely used antibiotic for the management of periodontitis, was the chosen model therapeutic agent to assess the effectiveness of this advanced drug delivery system.

Designed for use by dental healthcare professionals, this therapeutic approach is expected to be administered locally into periodontal and/or peri-implant pockets preferably using a specially designed, non-proprietary syringe equipped with a blunt needle. This design ensures a precise, safe, and ready-to-use application. This method of delivery is not only patient friendly but also optimizes the therapeutic potential of the chosen agent by concentrating the treatment directly at the site of need.

The effectiveness and innovation of this multifunctional system are underpinned by several key mechanisms and principles that set a new benchmark in periodontitis and/or peri-implantitis treatment:
- **Targeted local delivery:** The system introduces a direct, localized delivery of bioactive substances into periodontal pockets, focusing the treatment on affected areas to enhance efficacy and reducing systemic impact.
- **Enhanced uptake and cellular distribution:** Nanoscale size and surface properties ensure deep tissue penetration and effective system distribution across the cells of the local milieu.
- **Synergistic antibacterial activity at both intracellular and extracellular locations:** By exploiting the unique properties of zinc-magnesium layered hydroxide nanoparticles, the invention amplifies the activity of the therapeutic agents, offering synergistic effects that exceed those of conventional treatments, further accessing the intracellular environment.
- **pH-responsive release mechanism:** The system is engineered to release therapeutic agents faster in acidic conditions found in infected periodontal sites, delivering the agents precisely when and where needed.
- **Biocompatibility and bioactivity:** The nanoparticles are designed to be biocompatible, bioresorbable, and inherently bioactive, facilitating their seamless integration, uptake, and utilization by cells and tissues avoiding adverse effects. Their bioactivity is instrumental in modulating inflammation, oxidative stress and promoting the healing of periodontal and peri-implant tissues.
- **Improved patient compliance:** The ease of application, combined with decreased application frequency, potentially enhances patient comfort and adherence to treatment.

By integrating these mechanisms into a single, holistic treatment approach, this invention can offer a highly effective, patient-friendly, and environmentally sustainable adjunctive solution for managing periodontitis and/or peri-implantitis, promising enhanced treatment outcomes and a significant advancement in dental care.

### Brief description of drawings

For easier understanding of this application, figures are attached in the annex that represent the preferred forms of implementation which nevertheless are not intended to limit the technique disclosed herein.

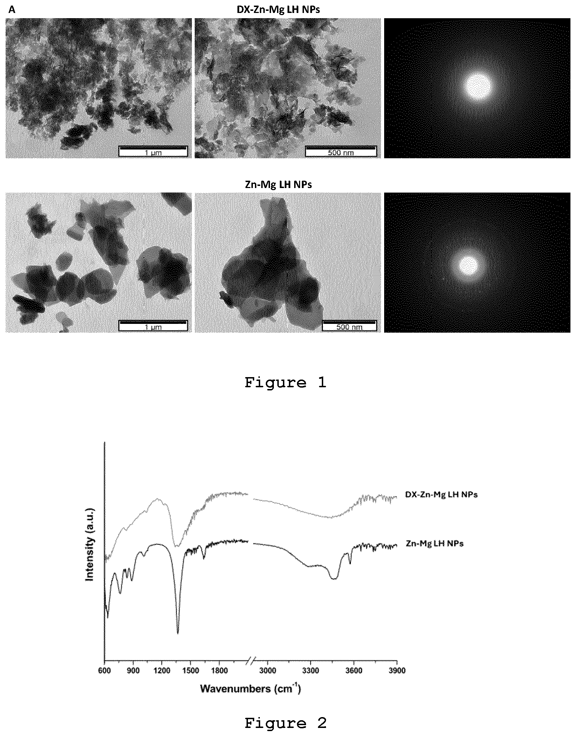

Figure 1 shows physicochemical characterization of zinc-magnesium layered hydroxide nanoparticles (Zn-Mg LH NPs) and doxycycline-loaded zinc-magnesium layered hydroxide nanoparticles (DX-Zn-Mg LH NPs). Nanoparticle's morphology, Selected area electron diffraction (SAED) and size were visualized by transmission electron microscopy (TEM) (Hitachi H-9000-NA), with an acceleration voltage of 200 kV, after coating the samples with a gold/palladium conductive layer.
Figure 2 shows physicochemical characterization of zinc-magnesium layered hydroxide nanoparticles (Zn-Mg LH NPs) and doxycycline-loaded zinc-magnesium layered hydroxide nanoparticles (DX-Zn-Mg LH NPs) by Fourier transform infrared (FTIR) with attenuated total reflectance (ATR) using a Nicolet (Thermo Electron) spectrometer with an attenuated total reflectance (ATR) apparatus ranging from 4000 cm⁻¹ to 600 cm⁻¹, resolution of 4 cm⁻¹ and 128 scans.

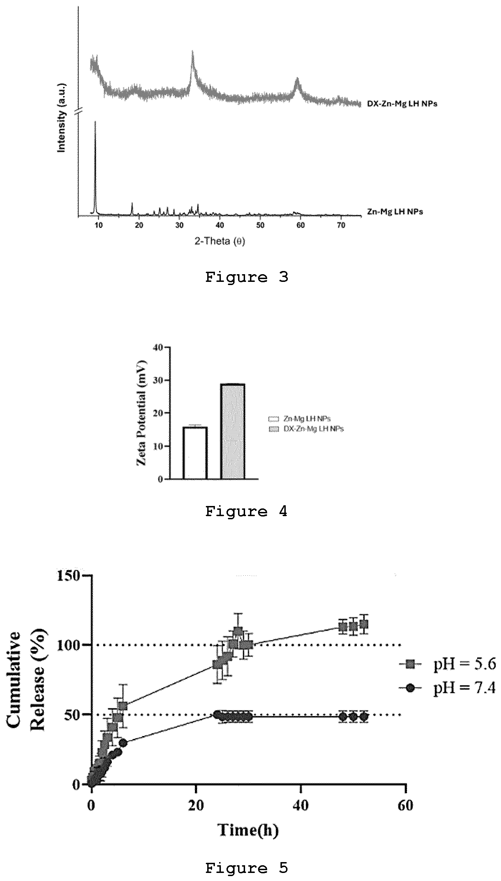

Figure 3 shows physicochemical characterization of zinc-magnesium layered hydroxide nanoparticles (Zn-Mg LH NPs) and doxycycline-loaded zinc-magnesium layered hydroxide nanoparticles (DX-Zn-Mg LH NPs) by X-ray diffraction (XRD) using a D8 Advance Bruker AXS (Bruker) powder diffractometer equipped with a SSD160 detector and a copper radiation source (Cu Kα, λ = 1.5406 Å), operated at 30 kV and 30 mA, considering the 2θ range from 5 to 70_{°}, using a step size of 0.05°.
Figure 4 shows Zeta potential evaluations of zinc-magnesium layered hydroxide nanoparticles (Zn-Mg LH NPs) and doxycycline-loaded zinc-magnesium layered hydroxide nanoparticles (DX-Zn-Mg LH NPs) relying on light scattering methodology, using Zetasizer Nanoseries Nano-Z (Malvern, Worcestershire, UK), resuspended in distilled water, at 25 °C.
Figure 5 shows doxycycline release at different pH values. Drug loading capacity, doxycycline (DX) spectrum and drug release profile of DX-Zn-Mg LH NPs were evaluated by spectrophotometry using a microplate reader (FLUOstar Omega, BMG Labtech, Germany). Drug release was performed using dialysis membranes (300 kDa, Spectra/Por, Float-A-Lyzer-G2) in HEPES buffer, under constant shacking at 37 °C. Quantification of doxycycline was set at 345 nm.

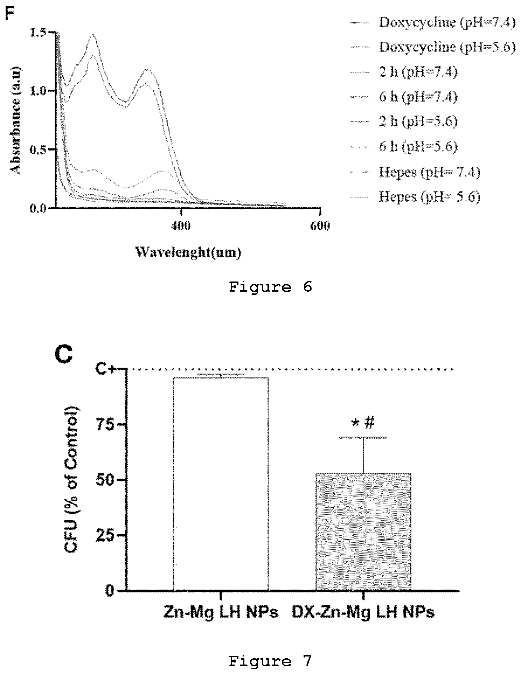

Figure 6 shows doxycycline spectrum after the release. Drug loading (%) was approx. 7.9%. The graph illustrates a sequential arrangement of lines, where the vertical positioning from top to bottom corresponds to Doxycycline solution (pH of 7.4), Doxycycline solution (pH of 5.6), drug release medium (6 h, pH of 5.6), drug release medium (6 h, pH of 7.4), drug release medium (2 h, pH of 5.6), drug release medium (2 h, pH of 7.4), Hepes (pH of 5.6), and Hepes (pH of 7.4).
Figure 7 shows Colony-forming unit (CFU) counting of *Porphyromonas gingivalis* (ATCC 33277), after 24 h incubation with the nanoparticles, at 10 µg/mL. (*) Statistically different to Control. (#) Statistically different to other experimental samples (p<0.05). A PBS suspension of nanoparticles at 10 ug/mL was incubated with 10⁶ CFUs mL⁻¹ of bacteria inoculum seeded on blood agar medium plates (Blood Agar Oxoid No 2), supplemented with 5% (v/v) sterile horse blood (Oxoid), 5.0 mg/L hemin (Sigma) and 1.0 mg/L menadione (Merck) for 24 h under anaerobic conditions at 37 °C, prior CFU counting.

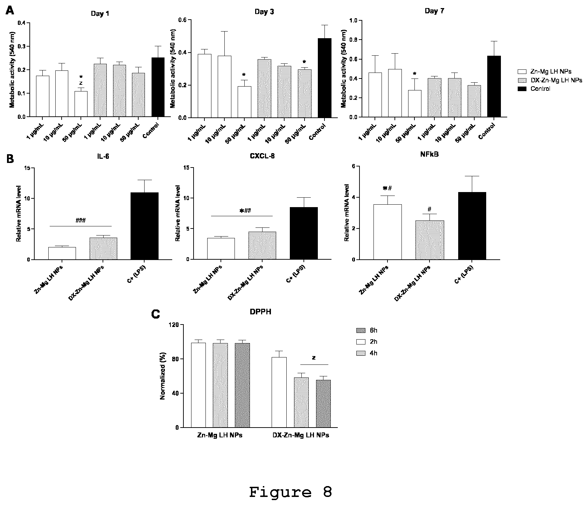

Figure 8 shows the biological characterization of layered hydroxide nanoparticles (LH NPs) using human gingival fibroblasts (HGFs, ATCC^{®} PCS-201-018^{™}) from the 9^{th} passage incubated at 37 °C and 5% CO₂ in air. A - The metabolic activity of HGFs incubated with DX-Zn-Mg LH and Zn-Mg LH NPs, assessed using the MTT assay. Cells were incubated for 3 h, at 37 °C. Formazan crystals were solubilized using dimethyl sulfoxide and absorbance of the final solution was read at 550 nm (Synergy HT; BioTek). B - Anti-inflammatory activity of DX-Zn-Mg LH and Zn-Mg LH NPs at 10 pg/mL. The organotypic mucosal model was stimulated with 1 µg/mL of *P*. *gingivalis'* lipopolysaccharide (LPS, from Sigma) for 16 h to induce an inflammatory phenotype, prior NPs' incubation, at 37 °C. After 24 h, The total RNA was isolated from the cell cultures using Trizol reagent (Ambion, Life technologies) following the manufacturer's protocol. The conversion of RNA to cDNA was performed using a reverse transcription system (NZY first-strand cDNA synthesis), where the concentration of the RNA was normalized to 400 ng/pL. Quantitative PCR (qPCR) was accomplished using the CFX384 real-time PCR system (Bio-Rad). Samples were prepared by adding cDNA, iTaq Universal SYBR green Supermix PCR Kit, DEPC water and the pre-defined primers from Bio-Rad. The relative expression of each gene was obtained by their normalization to GAPDH levels and further application of the 2^{-ΔΔCt} method. C - ROS scavenging activity of NPs was evaluated through the 2,2-diphenyl-1-picrylhydrazyl (DPPH, Sigma) assay. 10 mg of NPs were put in a 1.5 mL Epp and suspended in 500 µL of a DPPH-ethanolic solution at 0.1 mM at room temperature, under slow agitation (70 rpm). The absorbance of supernatants was read at 517 nm using a microplate reader (Synergy HT; BioTek). (*) Statistically different to Negative control; (#) Statistically different to Positive control; (z) Statistically different to other experimental samples (p<0.05).

### Detailed description of embodiments

Now, preferred embodiments of the present application will be described in detail with reference to the annexed drawings. However, they are not intended to limit the scope of this application.

The present application relates to zinc-magnesium layered hydroxide nanoparticles as a drug delivery system for the treatment of periodontitis and/or peri-implantitis.

In one embodiment, the zinc-magnesium layered hydroxide nanoparticles are loaded with at least one type of hydrophilic therapeutic agent, such as tetracycline antibiotics, suitable for the treatment of periodontitis and/or peri-implantitis. In one embodiment, the tetracycline antibiotic can be selected from doxycycline or minocycline.

The zinc-magnesium layered hydroxide nanoparticles have a plate-like morphology, with nano-scaled thickness, enhancing their bioactivity and cellular uptake.

The zinc-magnesium layered hydroxide nanoparticles have a structure made of hydroxide layers comprising an interlayer spacing between 9.5 to 10 Å and a layer thickness between 0.4 and 0.7 nm.

The zinc-magnesium layered hydroxide nanoparticles have a positive surface charge with values between 15 and 40 mV.

The zinc-magnesium layered hydroxide nanoparticles have a high crystallinity degree.

In one embodiment, a pharmaceutical composition comprises the zinc-magnesium layered hydroxide nanoparticles loaded with at least one type of hydrophilic therapeutic agent suitable for the treatment of periodontitis and/or peri-implantitis.

In one embodiment, the drug loading (%) of the hydrophilic therapeutic agent ranges between 7 and 15% in weight.

In one embodiment, the zinc-magnesium layered hydroxide nanoparticles loaded with at least one type of hydrophilic therapeutic agent or the pharmaceutical composition comprising the zinc-magnesium layered hydroxide nanoparticles loaded with at least one type of hydrophilic therapeutic agent are administered in the periodontal and/or peri-implant pockets with probing depth of ≥ 5 mm.

In one embodiment, the administering is performed by a healthcare dental medicine professionals once a week for a period ranging from 1 to 4 weeks.

In another aspect, the application discloses a method to obtain zinc-magnesium layered hydroxide nanoparticles as a drug delivery system for the treatment of periodontitis and/or peri-implantitis comprising the following steps:
- Preparing a zinc nitrate aqueous solution with a weight concentration between 25 to 32%;
- Preparing a magnesium nitrate aqueous solution with a weight concentration between 2 to 3%;
- Adding at least one type of hydrophilic therapeutic agent to the magnesium nitrate aqueous solution under stirring and a temperature between 20 and 25°C, for at least 5 minutes, obtaining a concentration between 1 to 5 mg/mL of the agent;
- Adding the zinc nitrate aqueous solution to the previous solution, obtaining a mixture with a Zn:Mg ratio ranging from 9:1 to 11:1 M;
- Adjusting the pH of the mixture between 1.5 and 2.5;
- Adding a precipitation agent dropwise to the mixture under stirring and temperature between 20 and 25°C, for at least 10 minutes;
- Adjusting the pH of the mixture between 5 and 6;
- Filtering the mixture under vacuum at a temperature between 20 and 25°C to obtain a precipitate;
- Washing the precipitate with ultra-pure water and drying the precipitate at a temperature between 20 and 25°C under vacuum for at least 24 h to obtain the nanoparticles loaded with the therapeutic agent.

In one embodiment, the drug loading (%) of the hydrophilic therapeutic agent ranges between 7 and 15% in weight.

In one embodiment, the ratio between NPs:therapeutic agent ranges between 5.7:1 and 13:1 weight.

In one embodiment, the zinc nitrate aqueous solution is obtained by dissolving zinc nitrate hexahydrate in ultra-pure water, under stirring 300 RPM at a temperature between 20 and 25°C, for at least 5 minutes.

In one embodiment, the magnesium nitrate aqueous solution is obtained by dissolving magnesium nitrate hexahydrate in ultra-pure water, under stirring 300 RPM at a temperature between 20 and 25°C, for at least 5 minutes.

In one embodiment, the precipitation agent is selected from, but not limited to, ammonia (aq) at 25%.

In one embodiment, stirring is carried out between 300 to 900 RPM, for 5 to 10 minutes.

In one embodiment, the filtering step is carried out with a 0.22 µm filter membrane.

### Examples:

The development and functionality of the zinc-magnesium layered hydroxide nanoparticles have been validated experimentally through designed investigation. Obtained data from *in vitro* experimental assessment indicate that the envisioned application performance requirements can be accomplished. The *in vitro* characterization of developed nanoparticles was set on three modalities - physicochemical
qualities, antibacterial activity, and cytocompatibility/bioactivity evaluation. Nanoparticle's physicochemical evaluation featured their morphology and size, visualized by transmission electron microscopy (Hitachi H-9000-NA), chemically, relying on Fourier transformed infrared spectroscopy (Nicolet, from Thermo Electron, with an attenuated total reflectance apparatus), crystallinity, assessed through X-ray diffraction (D8 Advance Bruker AXS, from Bruker), and zeta potential that was measured by light scattering (Zetasizer Nanoseries Nano-Z from Malvern), with nanoparticles suspended in distilled water. Drug loading capacity, doxycycline (DX) spectrum and drug release profile of DX-Zn-Mg LH NPs were quantified by spectrophotometry (FLUOstar Omega, BMG Labtech). Drug release was performed using dialysis membranes (300 kDa, Spectra/Por, Float-A-Lyzer-G2) in HEPES buffer at 37°C.

Antibacterial activity was assessed using *Porphyromonas gingivalis* (ATCC 33277), portraying gram-negative bacteria and periodontal pathogens. Methodologies such as colony-forming units counting were performed. Monocultures of human gingival fibroblasts (ATCC^{®} PCS-201-018) were used to evaluate nanoparticle's cytotoxicity through their effects on cellular metabolic activity (MTT assay), quantified by absorbance reading (Synergy HT; BioTek); anti-inflammatory effects on LPS-stimulated organotypic model of oral mucosa through quantitative gene expression, using a CFX384 real-time PCR system (Bio-Rad). NP's ROS scavenging capacity was evaluated using DPPH (Sigma), quantified by absorbance reading (Synergy HT; BioTek).

### 1. Method for obtaining zinc-magnesium layered hydroxide nanoparticles:

7.43 g of Zinc nitrate hexahydrate (Sigma) was dissolved in 25 mL of ultra-pure water under magnetic stirring, at 300 RPM, to obtain a zinc nitrate aqueous solution. 0.64 g of magnesium nitrate hexahydrate (Sigma) were dissolved in 25 mL of ultra-pure water under magnetic stirring, at 300 RPM, to obtain a magnesium nitrate aqueous solution. 125 mg of a hydrophilic therapeutic agent was added into the magnesium nitrate aqueous solution under magnetic stirring, at 300 RPM. The zinc nitrate aqueous solution was added to the antibiotic-magnesium nitrate aqueous solution under magnetic stirring, at 900 RPM. The pH value was maintained between 1.7 and 2.3. The Zn and Mg ratio in the mixture was 10:1 M. 1 mL of ammonia solution 25% (precipitation agent, Sigma) was added to the mixture under magnetic stirring at 900 RPM, maintained for 5 min, to promote precipitation of the zinc-magnesium layered hydroxide nanoparticles. Final pH value of the mixture should be between 5 to 6. The precipitate and the supernatant were poured onto a vacuum filtration unit with a 0.22 µm filter membrane (Sarstedt) attached. Supernatant was removed, while the precipitate stayed over the filter membrane. The precipitate was washed with ultra-pure water, three times, and placed the precipitate into a vacuum chamber at 25 °C. Once washed and dried, the precipitate made of the nanoparticles was ready for use or sterilization.

As observed in TEM analysis (Figure 1), Zn-Mg LH NPs exhibited well-defined particles with a characteristic two-dimensional sheet-like morphology. Moreover, the selected area electron diffraction (SAED) pattern showed well-defined rings corresponding to (200) and (400) planes with bright diffraction spots that revealed highly crystalline Zn-Mg LH NPs and confirmed the presence of a LH structure [1]. Comparatively, DX-Zn-Mg LH NPs seemed more dispersed, with a similar shape, smaller size and lower crystallinity degree. Both nanoparticles presented some translucency indicating a nano-scaled thickness.

The FTIR/ATR spectroscopy (Figure 2) of Zn-Mg LH NPs qualitatively confirmed the presence of hydroxyl groups in the hydroxide layers, characterized by a sharp band at 3572 cm⁻¹, with water molecules in-between, represented as a broad absorbance peak at 3448 cm⁻¹ and 1628 cm⁻¹. In addition, interlayered nitrate was noted at 1365 cm⁻¹ and 830 cm⁻¹, while Zn-OH vibration bands at 1008, 879, 764, 632 cm⁻¹ were observed, indicating the presence of zinc hydroxynitrate [2,3]. Furthermore, the presence of DX in the nanoparticle is indicated by the enlarged vibration band at 1359 cm⁻¹, being extended from 1311 to 1380 cm⁻¹, which can be assigned to δCO group vibrations [4]. Similarly, a signal broadening between 1600-1670 cm⁻¹ was also noted, which can be related to uCC vibrations in the phenyl ring [4].

Regarding the XRD analysis (Figure 3), Zn-Mg LH NPs presented multiple and intense reflection 2-theta (0) peaks, including peaks at 9, 18, 34 and 59°, corroborating that zinc hydroxynitrate Zn₅(NO₃)₂(OH)₈.2H₂O is the detected crystalline phase, organized in monoclinic layers [5,6]. Despite the decrease in the crystallinity degree, DX-Zn-Mg LH NPs retained these four reflection peaks, confirming the production of layered zinc hydroxide particulate. Moreover, the a-axis, perpendicular to the sheet surface, serves as the growth direction for the nanosheets. The calculated interlayer spacing of (200) is 9.79 Å, aligns with the literature data of 9.75 Å for hydrated LZH [7]. Considering the size of the nitrate ion (approximately 4 Å in diameter along the molecular plane) and the layer thickness LZH (0.48

nm) [8], it's plausible that the nitrate anions in Zn-Mg LH could adopt a vertical orientation, enabling the interlayer to accommodate one nitrate anion within it.

The surface charge of Zn-Mg LH NPs (Figure 4) exhibited a positive zeta potential in distilled water, which is likely attributed to the presence of Zn²⁺ ions that are incorporated into the forming layers [9]. This finding is aligned with the literature, where layered zinc hydroxide (LZH) materials displayed positive zeta potential values [10,11]. The observed increase in the zeta potential of DX-Zn-Mg LH NPs could be attributed to the presence of protonated and zwitterionic forms of doxycycline in pH values below 7.0 [12,13]. Additionally, this positively charged surface plays an important role in promoting the NPs' uptake, exploiting the negatively charged cell membranes [14,15]. As observed in figure 5 and 6, DX was successfully released from the LH NPs, confirming their potential as a drug carrier system. Similarly, LH NPs containing TCs displayed a burst release in the first hours [16-18], followed by a subsequent controlled release [16]. It's worth mentioning that acidic environments can hasten the dissolution of LH nanoparticles, ramping up the release of DX, as depicted in Figure 5 [19].

DX-Zn-Mg LH NPs were effective against *P. gingivalis* at cytocompatible concentrations (i.e., 10 pg/mL), corroborating their feasibility as a local drug delivery system and their safety for medical applications (Figure 7). Furthermore, it is possible that, even though Zn-Mg LH NPs lack bactericidal properties on their own, they might have enhanced DX's antibacterial effects. This trend is documented with distinct zinc-based NPs against other bacteria (e.g., *P. aeruginosa, E. coli, S. epidermidis*) due to the inhibition of bacterial transporter proteins, biofilm formation, and the reversal of antibiotic resistance mechanisms, by releasing a significant quantity of cations. [4,20,21] .

The cytocompatibility of the developed nanoparticles was assessed in human gingival fibroblast (HGF) cultures. As Figure 8A illustrates, Zn-Mg LH and DX-Zn-Mg LH NPs, at concentrations of 1.0 and 10 pg/mL, did not exhibit any significant changes in the HGF's metabolic activity. However, it was reduced by approximately 50% at 50 ug/mL compared to the control. The presence of DX in the NPs did not significantly influence the metabolic activity of HGFs. Despite Zn-based materials being considered safe, cytotoxicity in mammal cells is reported in the literature [22]. Zinc-based nanoparticles can act as pro-oxidants, leading to oxidative damage [19], and excessive Zn²⁺ can disturb the cellular homeostasis by impairing electron transport systems, enzymatic and mitochondrial activity [22-24] .

Lipopolysaccharide (LPS), a component of the cell wall of gram-negative bacteria such as *P. gingivalis,* is considered a primary pro-inflammatory compound. It interacts with cellular Toll-Like Receptor-4 (TLR4), leading to the production and secretion of pro-inflammatory cytokines, including IL-1β, IL-6, TNF, and CXCL-8, through the activation of the NFkB transcription factor [25,26]. Unsurprisingly, the exposure of the organotypic model of oral mucosa to LPS resulted in a heightened inflammatory response, as evidenced by the upregulation of all the inflammatory genes assessed (Figure 8B). The incubation of the LPS-stimulated mucosal model with either Zn-Mg LH or DX-Zn-Mg LH NPs downregulated the expression of pro-inflammatory genes, indicating their anti-inflammatory capability. Similar results can be found in the literature, as Zn-based NPs were able to reduce inflammatory markers within *in vitro* and *in vivo* settings, corroborating our findings [27-29]. This property can be attributed to the release of Zn²⁺, which downregulates the expression of proinflammatory cytokines such as IL-1β and IL-6 by preventing the activation of the NFKB pathway [30].

Lastly, only DX-Zn-Mg LH NPs exhibited ROS scavenging activity (Figure 8C). Tetracyclines are also known by their antioxidant properties [31]. Their phenolic rings can react with free radicals, forming stable and non-reactive molecules. This ultimately leads to the reduction of key oxidant markers, such as nitric oxide and protein carbonylation [32,33].

### References

1. Martin, V.; Bettencourt, A.F.; Fernandes, M.H.; Alves, M.M.; Hanafy, M.; Cui, Z.; Gomes, P.S.; Santos, C. Green Synthesis of Zn-Mg Layered Hydroxide Nanoparticles with Surface-Mediated Antioxidant and Anti-Inflammatory Activity. Surfaces and Interfaces 2024, 46, 104037, doi:10.1016/j.surfin.2024.104037.
2. Shinagawa, T.; Watanabe, M.; Mori, T.; Tani, J.I.; Chigane, M.; Izaki, M. Oriented Transformation from Layered Zinc Hydroxides to Nanoporous ZnO: A Comparative Study of Different Anion Types. Inorganic Chemistry 2018, 57, 13137-13149, doi:10.1021/acs.inorgchem.8b01242.
3. Li, P.; Xu, Z.P.; Hampton, M.A.; Vu, D.T.; Huang, L.; Rudolph, V.; Nguyen, A. V Control Preparation of Zinc Hydroxide Nitrate Nanocrystals and Examination of the Chemical and Structural Stability. The Journal of Physical Chemistry C 2012, 116, 10325-10332, doi:10.1021/jp300045u.
4. Rogowska, A.; Railean-plugaru, V.; Pomastowski, P.; Walczak-skierska, J.; Król-górniak, A.; Golebiowski, A.; Buszewski, B. The Study on Molecular Profile Changes of Pathogens via Zinc Nanocomposites Immobilization Approach. International Journal of Molecular Sciences 2021, 22, doi:10.3390/ijms22105395.
5. Demel, J.; Hynek, J.; Kovář, P.; Dai, Y.; Taviot-Guého, C.; Demel, O.; Pospíšil, M.; Lang, K. Insight into the Structure of Layered Zinc Hydroxide Salts Intercalated with Dodecyl Sulfate Anions. *The Journal of Physical Chemistry C* **2014,** *118*, 27131-27141, doi:10.1021/jp508499g.
6. Newman, S.P.; Jones, W. Comparative Study of Some Layered Hydroxide Salts Containing Exchangeable Interlayer Anions. J Solid State Chem 1999, 148, 26-40, doi:10.1006/jssc.1999.8330.
7. Ruiz, C. V.; Rodríguez-Castellón, E.; Giraldo, O. Structural Analysis and Conduction Mechanisms in Polycrystalline Zinc Hydroxide Nitrate. Inorg Chem 2018, 57, 9067-9078, doi:10.1021/acs.inorgchem.8b01074.
8. Saifullah, B.; Arulselvan, P.; El Zowalaty, M.E.; Fakurazi, S.; Webster, T.J.; Geilich, B.; Hussein, M.Z. Development of a Highly Biocompatible Antituberculosis Nanodelivery Formulation Based on Para-Aminosalicylic Acid - Zinc Layered Hydroxide Nanocomposites. Scientific World Journal 2014, 2014, doi:10.1155/2014/401460.
9. Ruiz, C. V.; Becerra, M.E.; Giraldo, O. Structural, Thermal, and Release Properties of Hybrid Materials Based on Layered Zinc Hydroxide and Caffeic Acid. Nanomaterials 2020, 10, doi:10.3390/nano10010163.
10. Saifullah, B.; Hussein, M.Z.; Hussein-Al-Ali, S.H.; Arulselvan, P.; Fakurazi, S. Sustained Release Formulation of an Anti-Tuberculosis Drug Based on Para-Amino Salicylic Acid-Zinc Layered Hydroxide Nanocomposite. Chem Cent J 2013, 7, 72, doi:10.1186/1752-153X-7-72.
11. Nabipour, H.; Batool, S.; Hu, Y. Pectin-Coated Baclofen-Layered Zinc Hydroxide Nanohybrid as a Bio-Based Nanocomposite Carrier for Oral Delivery. IEEE Trans Nanobioscience 2023, 22, 63-70, doi:10.1109/TNB.2022.3155785.
12. Debnath, B.; Majumdar, M.; Bhowmik, M.; Bhowmik, K.L.; Debnath, A.; Roy, D.N. The Effective Adsorption of Tetracycline onto Zirconia Nanoparticles Synthesized by Novel Microbial Green Technology. J Environ Manage 2020, 261, doi:10.1016/j.jenvman.2020.110235.
13. Gopal, G.; Sankar, H.; Natarajan, C.; Mukherjee, A. Tetracycline Removal Using Green Synthesized Bimetallic NZVI-Cu and Bentonite Supported Green NZVI-Cu Nanocomposite: A Comparative Study. J Environ Manage 2020, 254, doi:10.1016/j.j envman.2019.109812.
14. Hu, T.; Gu, Z.; Williams, G.R.; Strimaite, M.; Zha, J.; Zhou, Z.; Zhang, X.; Tan, C.; Liang, R. Layered Double Hydroxide-Based Nanomaterials for Biomedical Applications. Chem Soc Rev 2022, 51, 6126-6176, doi:10.1039/d2cs00236a.
15. Martin, V.; Francisca Bettencourt, A.; Santos, C.; Sousa Gomes, P. Reviewing Particulate Delivery Systems Loaded with Repurposed Tetracyclines - From Micro to Nanoparticles. Int J Pharm 2024, 649, 123642, doi:10.1016/j.ijpharm.2023.123642.
16. Chakraborti, M.; Jackson, J.K.; Plackett, D.; Gilchrist, S.E.; Burt, H.M. The Application of Layered Double Hydroxide Clay (LDH)-Poly(Lactide-Co- Glycolic Acid) (PLGA) Film Composites for the Controlled Release of Antibiotics. Journal of Materials Science: Materials in Medicine 2012, 23, 1705-1713, doi:10.1007/s10856-012-4638-y.
17. Chahardahmasoumi, S.; Sarvi, M.N.; Jalali, S.A.H. Modified Montmorillonite Nanosheets as a Nanocarrier with Smart PH-Responsive Control on the Antimicrobial Activity of Tetracycline upon Release. Applied Clay Science 2019, 178, 105135, doi:10.1016/j.clay.2019.105135.
18. Bouaziz, Z.; Soussan, L.; Janot, J.-M.; Jaber, M.; Ben Haj Amara, A.; Balme, S. Dual Role of Layered Double Hydroxide Nanocomposites on Antibacterial Activity and Degradation of Tetracycline and Oxytetracyline. Chemosphere 2018, 206, 175-183, doi:10.1016/j.chemosphere.2018.05.003.
19. Liao, C.; Jin, Y.; Li, Y.; Tjong, S.C. Interactions of Zinc Oxide Nanostructures with Mammalian Cells: Cytotoxicity and Photocatalytic Toxicity. International Journal of Molecular Sciences 2020, 21, 6305, doi:10.3390/ijms21176305.
20. Pandey, P.; Sahoo, R.; Singh, K.; Pati, S.; Mathew, J.; Pandey, A.C.; Kant, R.; Han, I.; Choi, E.H.; Dwivedi, G.R.; et al. Drug Resistance Reversal Potential of Nanoparticles/ Nanocomposites via Antibiotic's Potentiation in Multi Drug Resistant p. Aeruginosa. Nanomaterials 2022, 12, doi:10.3390/nano12010117.
21. El-Ela, F.I.A.; Farghali, A.A.; Mahmoud, R.K.; Mohamed, N.A.; Moaty, S.A.A. New Approach in Ulcer Prevention and Wound Healing Treatment Using Doxycycline and Amoxicillin/LDH Nanocomposites. Scientific Reports 2019, 9, 6418, doi:10.1038/s41598-019-42842-2.
22. Król, A.; Pomastowski, P.; Rafińska, K.; Railean-Plugaru, V.; Buszewski, B. Zinc Oxide Nanoparticles: Synthesis, Antiseptic Activity and Toxicity Mechanism. *Advances in Colloid and Interface Science* **2017,** *249*, 37-52, doi:10.1016/j.cis.2017.07.033.
23. Chang, M.-C.; Tang, C.; Lin, Y.-H.; Liu, H.; Wang, T.; Lan, W.; Cheng, R.; Lin, Y.; Chang, H.; Jeng, J.-H. Toxic Mechanisms of Roth801, Canals, Microparticles and Nanoparticles of ZnO on MG-63 Osteoblasts. Materials Science and Engineering: C 2021, 119, 111635, doi:10.1016/j.msec.2020.111635.
24. Shen, C.; James, S.A.; de Jonge, M.D.; Turney, T.W.; Wright, P.F.A.; Feltis, B.N. Relating Cytotoxicity, Zinc Ions, and Reactive Oxygen in ZnO Nanoparticle-Exposed Human Immune Cells. Toxicological Sciences 2013, 136, 120-130, doi:10.1093/toxsci/kft187.
25. Nativel, B.; Couret, D.; Giraud, P.; Meilhac, O.; D'Hellencourt, C.L.; Viranaïcken, W.; Da Silva, C.R. Porphyromonas Gingivalis Lipopolysaccharides Act Exclusively through TLR4 with a Resilience between Mouse and Human. Scientific Reports 2017, 7, 1-12, doi:10.1038/s41598-017-16190-y.
26. Martin, V.; Ribeiro, I.A.C.; Alves, M.M.; Gon 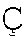 alves, L.; Almeida, A.J.; Grenho, L.; Fernandes, M.H.; Santos, C.F.; Gomes, P.S.; Bettencourt, A.F. Understanding Intracellular Trafficking and Anti-Inflammatory Effects of Minocycline Chitosan-Nanoparticles in Human Gingival Fibroblasts for Periodontal Disease Treatment. *International Journal of Pharmaceutics* **2019,** *572*, 118821, doi:10.1016/j.ijpharm.2019.118821.
27. Gad, S.S.; Fayez, A.M.; Abdelaziz, M.; Abou El-ezz, D. Amelioration of Autoimmunity and Inflammation by Zinc Oxide Nanoparticles in Experimental Rheumatoid Arthritis. Naunyn-Schmiedeberg's Archives of Pharmacology 2021, 394, 1975-1981, doi:10.1007/s00210-021-02105-2.
28. Lee, J.-H.; Lee, H.; Kim, K.-N.; Kim, K. Cytotoxicity and Anti-Inflammatory Effects of Zinc Ions and Eugenol during Setting of ZOE in Immortalized Human Oral Keratinocytes Grown as Three-Dimensional Spheroids. Dental Materials 2016, 32, e93-e104, doi:10.1016/j.dental.2016.01.003.
29. Hasannasab, M.; Nourmohammadi, J.; Dehghan, M.M.; Ghaee, A. Immobilization of Bromelain and ZnO Nanoparticles on Silk Fibroin Nanofibers as an Antibacterial and Anti-Inflammatory Burn Dressing. International Journal of Pharmaceutics 2021, 610, 121227, doi:10.1016/j.ijpharm.2021.121227.
30. Jarosz, M.; Olbert, M.; Wyszogrodzka, G.; Mlyniec, K.; Librowski, T. Antioxidant and Anti-Inflammatory Effects of Zinc. Zinc-Dependent NF-KB Signaling. Inflammopharmacology 2017, 25, 11-24, doi:10.1007/s10787-017-0309-4.
31. Golub, L.M.; Elburki, M.S.; Walker, C.; Ryan, M.; Sorsa, T.; Tenenbaum, H.; Goldberg, M.; Wolff, M.; Gu, Y. Non-Antibacterial Tetracycline Formulations: Host-Modulators in the Treatment of Periodontitis and Relevant Systemic Diseases. International Dental Journal 2016, 66, 127-135, doi:10.1111/idj.12221.
32. Altoé, L.S.; Alves, R.S.; Miranda, L.L.; Sarandy, M.M.; Bastos, D.S.S.; Gonçalves-Santos, E.; Novaes, R.D.; Gon 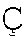 alves, R. V. Doxycycline Hyclate Modulates Antioxidant Defenses, Matrix Metalloproteinases, and COX-2 Activity Accelerating Skin Wound Healing by Secondary Intention in Rats. *Oxidative Medicine and Cellular Longevity* **2021,** *2021,* doi:10.1155/2021/4681041.
33. Garrido-Mesa, J.; Adams, K.; Galvez, J.; Garrido-Mesa, N. Repurposing Tetracyclines for Acute Respiratory Distress Syndrome (ARDS) and Severe COVID-19: A Critical Discussion of Recent Publications. Expert Opinion on Investigational Drugs 2022, 31, 475-482, doi:10.1080/13543784.2022.2054325.

## Claims

1. Zinc-magnesium layered hydroxide nanoparticles as a drug delivery system loaded with at least one type of hydrophilic therapeutic agent for the treatment of periodontitis and/or peri-implantitis, wherein the nanoparticles have a structure made of hydroxide layers comprising an interlayer spacing between 9.5 to 10 Å, a layer thickness between 0.4 and 0.7 nm, and a positive surface charge between 15 and 40mV.

2. The Zinc-magnesium layered hydroxide nanoparticles according to the previous claim, wherein the hydrophilic therapeutic agent is a tetracycline antibiotic.

3. The Zinc-magnesium layered hydroxide nanoparticles according to any of the previous claim, wherein the ratio between nanoparticles:therapeutic agent ranges between 5.7:1 and 13:1 weight.

4. The Zinc-magnesium layered hydroxide nanoparticles according to any of the previous claims, wherein the drug loading of the hydrophilic therapeutic agent ranges between 7 and 15% in weight.

5. Method to obtain the zinc-magnesium layered hydroxide nanoparticles disclosed in any of the previous claims, wherein it comprises the following steps:
- Preparing a zinc nitrate aqueous solution with a weight concentration between 25 to 32%;
- Preparing a magnesium nitrate aqueous solution with a weight concentration between 2 to 3%;
- Adding at least one type of hydrophilic therapeutic agent to the magnesium nitrate aqueous solution under stirring and a temperature between 20 and 25°C, for at least 5 minutes, obtaining a concentration between 1 to 5 mg/mL of the agent;
- Adding the zinc nitrate aqueous solution to the previous solution, obtaining a mixture with a Zn:Mg ratio ranging from 9:1 to 11:1 M;
- Adjusting the pH of the mixture between 1.5 and 2.5;
- Adding a precipitation agent dropwise to the mixture under stirring and temperature between 20 and 25°C, for at least 10 minutes;
- Adjusting the pH of the mixture between 5 and 6;
- Filtering the mixture under vacuum at a temperature between 20 and 25°C to obtain a precipitate;
- Washing the precipitate with ultra-pure water and drying the precipitate at a temperature between 20 and 25°C under vacuum for at least 24 h to obtain the nanoparticles loaded with the therapeutic agent.

6. Method according to the previous claim, wherein the zinc nitrate aqueous solution is obtained by dissolving zinc nitrate hexahydrate in ultra-pure water, under stirring 300 RPM at a temperature between 20 and 25°C, for at least 5 minutes.

7. Method according to any of the claims 5 to 6, wherein the magnesium nitrate aqueous solution is obtained by dissolving magnesium nitrate hexahydrate in ultra-pure water, under stirring 300 RPM at a temperature between 20 and 25°C, for at least 5 minutes.

8. Method according to any of the claims 5 to 7, wherein the precipitation agent is ammonia at 25%.

9. Method according to any of the claims 5 to 8, wherein stirring is carried out between 300 to 900 RPM.

10. Method according to any of the claims 5 to 9, wherein the filtering step is carried out with a 0.22 µm filter membrane.
